**Europäisches Patentamt**

(19) **European Patent Office**

**Office européen des brevets**

(11) Veröffentlichungsnummer. **0 026 485**
**B1**

(12) **EUROPÄISCHE PATENTSCHRIFT**

(45) Veröffentlichungstag der Patentschrift:
**19.09.84**

(21) Anmeldenummer: **80105837.1**

(22) Anmeldetag: **26.09.80**

(51) Int. Cl.³: **C 07 G 11/00**, C 12 P 1/04,
C 12 P 21/00, A 61 K 35/74 //
(C12P21/00, 1/04, C12R1/18)

(54) Herbicolin, Verfahren zu seiner Herstellung und es enthaltende Mittel.

(30) Priorität: **26.09.79 DE 2938993**

(43) Veröffentlichungstag der Anmeldung:
**08.04.81 Patentblatt 81/14**

(45) Bekanntmachung des Hinweises auf die Patenterteilung:
**19.09.84 Patentblatt 84/38**

(84) Benannte Vertragsstaaten:
**AT CH DE FR GB IT LI SE**

(56) Entgegenhaltungen:
**FR - A - 2 405 265**

**THE JOURNAL OF ANTIBIOTICS, Band XXXIII, Nr. 4,
April 1980, Seiten 353-358 Ausg. Japan Antibiotics
Research Association Tübingen G. WINKELMANN et al.:
"Herbicolins-New peptide antibiotics from Erwinia
Herbicola"**

(73) Patentinhaber: **Winkelmann, Günther, Dr. Prof.,
Märchenseestrasse 5,
D-7407 Rottenburg 4-Wendelsheim (DE)**

(72) Erfinder: **Winkelmann, Günther, Dr. Prof.,
Märchenseestrasse 5,
D-7407 Rottenburg 4-Wendelsheim (DE)**

(74) Vertreter: **Kraus, Walter, Dr. et al, Patentanwälte Kraus
& Weisert Irmgardstrasse 15, D-8000 München 71 (DE)**

## Beschreibung

Die vorliegende Erfindung betrifft ein neues Antibiotikum und Antimykotikum Herbicolin, ein mikrobiologisches Verfahren zu seiner Herstellung aus einem Stamm von Erwinia herbicola sowie ein es enthaltendes Mittel.

Sowohl in der Medizin als auch im Pflanzenschutz besteht nach wie vor ein Bedarf an hochwirksamen antibakteriellen und antimykotisch oder antifungisch wirksamen Substanzen.

Es wurde nun gefunden, daß man das neue Antibiotikum und Antimykotikum »Herbicolin« durch aerobes Züchten von Mikroorganismen der Ordnung Eubacteriales, speziell der Familie Enterobacteriaceae, insbesondere von Stämmen der Gattung Erwinia, in wäßrigem Medium und anschließende Isolierung gewinnen kann. Das neue Herbicolin besteht aus zwei chromatographisch trennbaren Komponenten, dem Herbicolin A und dem Herbicolin B, wobei das Herbicolin A in einer Menge von etwa 80% und das Herbicolin B in einer Menge von etwa 20% in Herbicolin enthalten sind.

Ferner wurde gefunden, daß das neue Antibiotikum Herbicolin eine starke antifungische Wirkung gegen zahlreiche Pilze aufweist, während es gegenüber Bakterien allgemein unwirksam ist.

Gegenstand der Erfindung ist Herbicolin A und Herbicolin B, erhalten durch Züchtung von Erwinia herbicola (A 111) (DSM. Nr. 1619) unter aeroben Bedingungen in einem Kohlenstoffquellen und Stickstoffquellen enthaltenden Kulturmedium und Gewinnung des Antibiotikums.

Die Erfindung betrifft weiterhin Herbicolin A, das gekennzeichnet ist durch

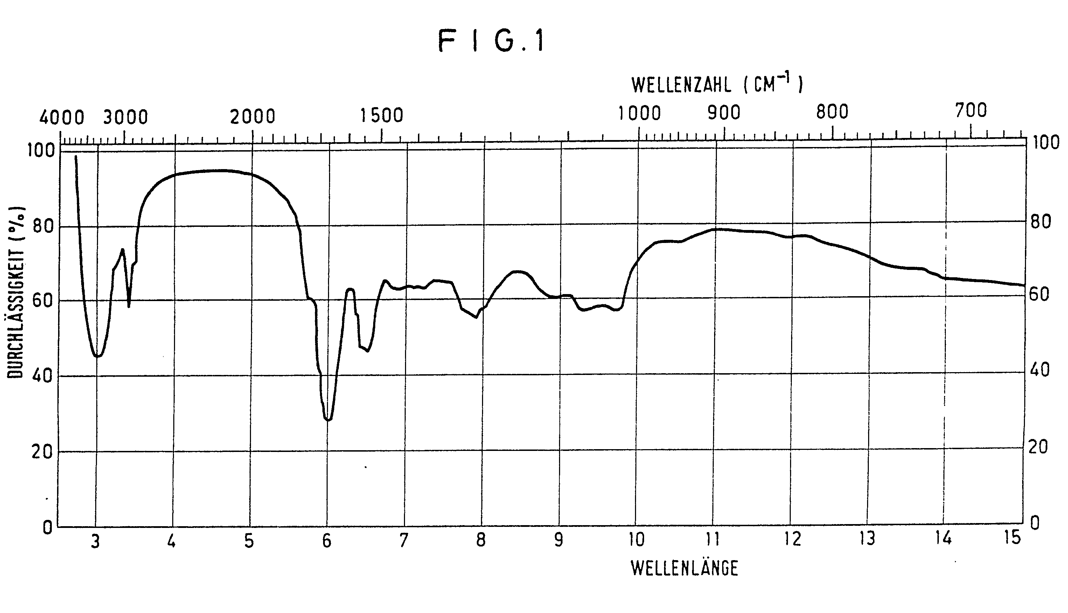

1) charakteristische IR-Banden in Kaliumbromid (Fig. 1) bei 3200, 2900, 2830, 1730, 1650, 1530 cm$^{-1}$

2) Löslichkeit:
sehr gut löslich in
    Methanol
    Äthanol
    Propanol-(1)
    Butanol-(1)
gut löslich in
    Äthylenglycol
    Äthylenglycolmonomethyläther
    Glycerin
    Wasser
gering löslich in
    Aceton
    Essigester
    Acetonitril
nahezu unlöslich in
    Chloroform
    Dichlormethan
    Diäthyläther
    Petroläther

wobei unter »gut löslich« verstanden wird, daß für die Erzielung der biologischen Wirkung genügend Herbicolin A in Lösung vorhanden ist,

3) Elementaranalyse:
gef.: C 46,1 H 7,1 N 11,8 S 1,9 Molmasse 1500 bis 3000

4) Charakteristische Reaktionen:
Ninhydrin          negativ
Chlor/4,4'-Tetramethyldiamin-
diphenylmethan    positiv

5) keine Absorption in UV zwischen 220 und 350 nm

6) nachweisbare Aminosäuren:

| Aminosäuren | Verhältnis (Näherung) |
|---|---|
| Glycin | 2 |
| L-Threonin | 1 |
| D-allo-Threonin | 1 |
| D-Glutaminsäure | 1 |
| D-Leucin | 1 |
| L-Arginin | 1 |

6a) $\beta$-Hydroxymyristinsäure

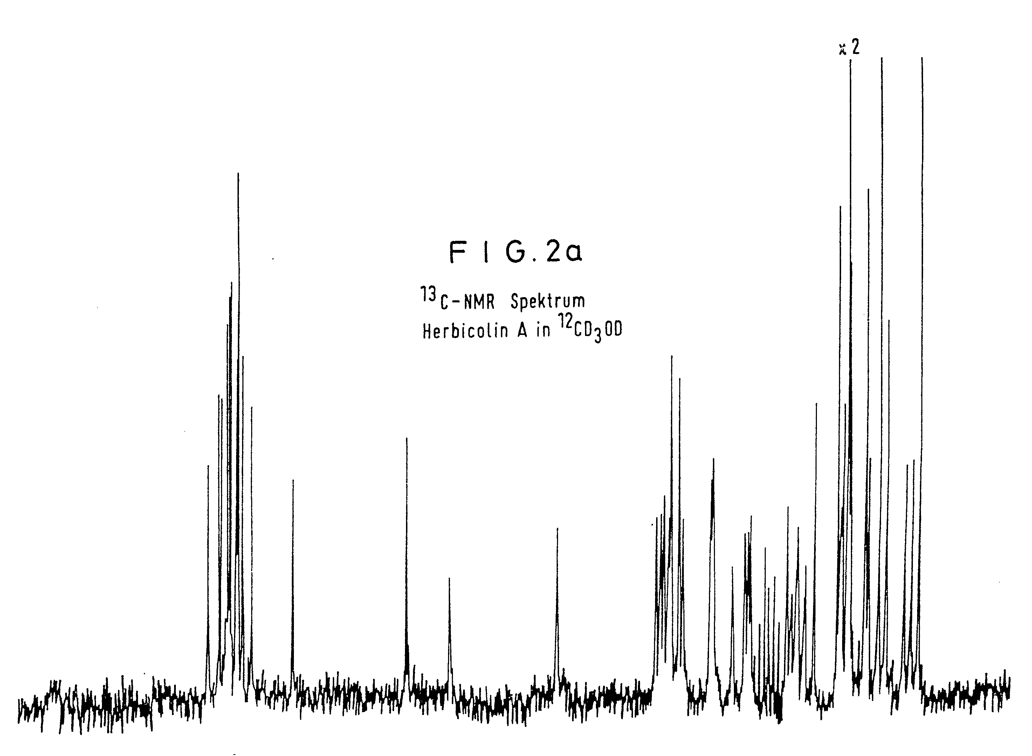

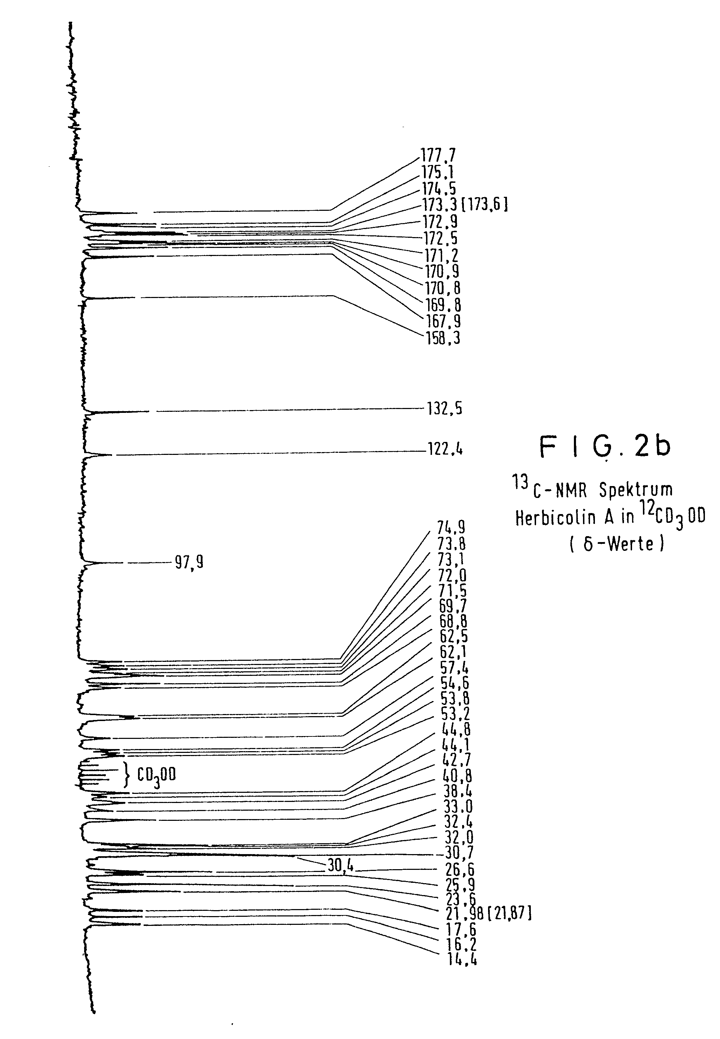

7) $^{13}$C-NMR Spektrum (Fig. 2a und 2b) mit ppm ($\delta$)-Werten in $^{12}$CD$_3$OD von 177,7, 175,1, 174,5, 173,3 [173,6], 172,9, 172,5 171,2, 170,9, 170,8, 169,8, 167,9, 158,3, 132,5, 122,4, 97,9, 74,9, 73,8, 73,1, 72,0, 71,5, 69,7, 68,8, 62,5, 61,2, 57,4, 54,6, 53,8, 53,2, 44,8, 44,1, 42,7, 40,8, 38,4, 33,0, 32,4, 32,0, 30,7, 30,4, 26,6, 25,9, 23,6, 21,98 [21,87], 17,6, 16,2 und 14,4

8) folgende Rf-Werte:
Butanol-(1)/Eisessig/Wasser
(4 : 1 : 1)        = Rf 0,06
Chloroform/Methanol/Eisessig/Wasser
(65 : 25 : 3 : 4)    = Rf 0,10

9) Wirksamkeit: starke fungitoxische Wirkung bei Konzentrationen von 0,1 bis 1 µg/ml Parameciumarten (Pantoffeltierchen) und Amöben werden in Konzentrationen von 1 bis 2 µg/ml in wenigen Sekunden abgetötet Erythrozyten werden in Konzentrationen von ca. 10 µg/ml (Humanblut) lysiert.

Die Erfindung betrifft weiterhin Herbicolin B, gekennzeichnet durch

a) charakteristische Reaktionen:
Chlor/4,4'-Tetramethyldiamindiphenyl-
methan positiv

b) Dünnschichtchromatographie auf Kieselgelplatten:
Butanol-(1)/Eisessig/Wasser
(4 : 1 : 1)        = Rf 0,19

Die Erfindung betrifft weiterhin ein Verfahren zur Herstellung von Herbicolin, das dadurch gekennzeichnet ist, daß man Erwinia herbicola (A 111) (DSM Nr. 1619) unter aeroben Bedingungen in einem Kohlenstoffquellen und Stickstoffquellen enthaltenden Kulturmedium züchtet und die Kulturlösung durch Zentrifugieren von den Bakterien befreit, das Zentrifugat auf eine Servachrom-XAD®-Säule gibt, mit entionisiertem Wasser wäscht, zur Entfernung von Pigmenten und anderen lipophilen Komponenten mit Aceton/Wasser (1 : 1) oder Lösungsmittelgemischen ähnlicher Polarität wäscht und dann das gewünschte Herbicolin mit Methanol oder einem Lösungsmittel ähnlicher Polarität eluiert und das erhaltene Eluat gegebenenfalls zur Gewinnung von Herbicolin eindampft und gegebenenfalls gefriertrocknet.

Die Erfindung betrifft weiterhin antibiotische und antimykotische Mittel, die dadurch gekennzeichnet sind, daß sie Herbicolin sowie übliche Trägerstoffe und Verdünnungsmittel oder Herbicolin A sowie übliche Trägerstoffe und Verdünnungsmittel oder Herbicolin B sowie übliche Trägerstoffe und Verdünnungsmittel enthalten.

Herbicolin, Herbicolin A und Herbicolin B sind wirksame antibiotische und antimykotische Mittel. Herbicolin A und Herbicolin B unterscheiden sich in ihrer Wirksamkeit nicht oder nur geringfügig.

Das Herbicolin A übertrifft in seiner Wirkung eine Reihe bekannter antifungischer Substanzen. Die minimalen Hemmkonzentrationen (MHK) liegen bei 0,1 bis 1,0 µg/ml. Herbicolin ist gegen mycelbildende und hefeartige Pilze wirksam und kann sowohl gegen phytopathogene Pilze als auch gegen Dermatophyten eingesetzt werden. Andererseits stellt das Herbicolin keine schwer abbaubare Verbindung dar, so daß Umweltschäden nicht zu erwarten sind.

Herbicolin wird durch submerse Kultur von geeigneten Mikroorganismen in geeigneten Nährlösungen unter geeigneten physikalischen Bedingungen erzeugt. Es kann aus der Kulturlösung durch Extraktion oder Adsorption abgetrennt und durch weitere geeignete Methoden angereichert werden.

Für die Gewinnung von Herbicolin kann der neue Stamm Erwinia herbicola (A 111) aus der Ordnung Eubacteriales, Familie Enterobacteriaceae, Gattung Erwinia, eingesetzt werden. Dieser Stamm wurde 1977 in Tübingen aus einem Oberflächengewässer isoliert. Er wurde unter der Nr. DSM 1619 am 13. August 1979 bei der Deutschen Sammlung für Mikroorganismen in Göttingen hinterlegt. Der Stamm wurde am Bakteriologischen Institut der Südd. Versuchs- und Forschungsanstalt für Milchwirtschaft als Erwinia herbicola identifiziert und ist durch folgende Eigenschaften gekennzeichnet:

1)  Morphologie:
    schlanke Stäbchen, Länge: 2,1 bis 3,5 µm
    lebhaft beweglich; peritrich begeißelt

2)  Biochemische Charakterisierung:

| | |
|---|---|
| Gram | — |
| Oxidase | — |
| Katalase | + |
| O/F-Test | fermentatives Wachstum mit Säurebildung |
| VP | + |
| Indol | — |
| H₂S | — |
| Inosit | + |
| Phe-desaminase | + |
| Orn | — |
| Lys | — |
| Urease | + |
| Arabinose | +/— |
| Rhamnose | — |
| Dextrose | + (ohne Gas) |
| Gelatine verflüss. | + |

Die zusammengefaßten Bestimmungsmerkmale identifizieren den Stamm (A 111) als zur Art Erwinia herbicola gehörend.

Für das erfindungsgemäße Verfahren zur Herstellung von Herbicolin verwendet man Nährmedien, die die üblichen Kohlenstoff- und Stickstoffquellen und die notwendigen Salze enthalten. Als Kohlenstoffquelle können verwendet werden: Kohlenhydrate, insbesondere Monosaccharide, wie z. B. Glucose oder Fructose, aber auch Disaccharide, wie z. B. Maltose oder Saccharose. Daneben können natürliche Gemische, wie z. B. Hefe/Malz-Extrakte verwendet werden. Als Stickstoffquelle können assimilierbare Stickstoffquellen, wie Maisquellflüssigkeit, Sojabohnenpulver, Pepton, Fleischextrakte, Hefeextrakte, Aminosäure, wie z. B. Asparagin, oder anorganische Verbindungen, wie z. B. Ammoniumsulfat oder Ammoniumchlorid, verwendet werden.

Verschiedene anorganische Salze, wie Natriumchlorid, Kaliumchlorid, Magnesiumsulfat, Kaliumhydrogenphosphat oder Kaliumdihydrogenphosphat, werden gegebenenfalls verwendet.

Als Hilfsstoffe können Antischaummittel eingesetzt werden, wie Polyole oder Silikone. Zur Einhaltung eines gewünschten pH-Bereichs werden Puffer, z. B. anorganische Phosphate, eingesetzt.

Bei der Durchführung des erfindungsgemäßen Verfahrens wird unter aeroben Bedingungen gearbeitet, z. B. unter Verwendung von Schüttelkulturen oder belüfteten Fermenterkulturen. Die Prozentverhältnisse der Nährlösungsbestandteile können in weiten Bereichen schwanken, im allgemeinen machen die Kohlenstoffquellen 1 bis 10%, vorzugsweise 2 bis 3%, aus, die Stickstoffquellen 0,1 bis 0,5%, vorzugsweise 0,2%, aus. Die Salze liegen in den üblichen Konzentrationen vor, d. h. im Bereich zwischen 0,01 bis 1 Gew.-%. Verdünnungsmittel für die Nährmedien ist entionisiertes Wasser oder Leitungswasser.

Zur Sterilisation sind die erforderlichen Bedingungen anzuwenden: Bei kleineren Volumen 20 min bei 121°C, bei größeren Volumen 30 min bei 134° C. Die pH-Werte der wachsenden Kulturen liegen bei 5,5 bis 8, vorzugsweise bei pH 7. Die Züchtungstemperatur kann zwischen 4 und 30° C, vorzugsweise bei 22 bis 25° C, liegen. Das Maximum der Antibiotikumproduktion wird mit Hilfe des Plattendiffusionstests und Neurospora crassa als Testkeim direkt aus dem Kulturenzentrifugat bestimmt.

Zur Aufarbeitung des Herbicolins wird die Kulturlösung durch Zentrifugation von den Bakterien befreit. Das Zentrifugat wird über eine Servachrom-XAD-Säule® (Serva Feinbiochemica GmbH & Co., Heidelberg) (= Adsorptionsmittel auf Polystyrolbasis) gegeben und mit 2 bis 3 Säulenvolumen entionisiertem Wasser gewaschen. Dabei bleibt das Herbicolin zusammen mit anderen lipophilen Komponenten auf der Säule adsorbiert. Durch eine nachfolgende Elution mit Aceton/Wasser (1 : 1) oder Lösungsmittelgemischen ähnlicher Polarität werden Pigmente und andere schwach liphophile Komponenten entfernt.

Durch Elution mit reinem Methanol, Äthanol, Propanol oder anderen Lösungsmitteln ähnlicher Polarität erhält man dann eine nur noch schwach gelb gefärbte methanolische Lösung von Herbicolin mit wenigen lipophilen Begleitkomponenten. Man erhält hier immer ein Gemisch aus Herbicolin A und Herbicolin B. Vorzugsweise verwendet man Methanol.

Die erhaltene Lösung, vorzugsweise eine methanolische Lösung, kann in an sich bekannter Weise eingedampft und/oder gefriergetrocknet werden. Dabei erhält man das Herbicolin, das zu ca. 80% aus Herbicolin A und ca. 20% aus Herbicolin B besteht.

Zur weiteren Reinigung und insbesondere zur Trennung des Herbicolins in Herbicolin A und Herbicolin B kann die bei der Chromatographie erhaltene Lösung, vorzugsweise methanolische Lösung, einer Gelfiltration an Sephadex LH 20® (Deutsche Pharmacia GmbH, Freiburg) in Methanol oder einem Lösungsmittel ähnlicher Polarität, wie Äthanol, Propanol, oder einer Trennung an Sephacryl 200® (Deutsche Pharmacia GmbH, Freiburg) oder an Biogel P 4® (BIO-RAD Laboratories GmbH, München) in Methanol/Wasser (1 : 1) oder Aceton/Wasser (1 : 1) oder Lösungsmitteln ähnlicher Polarität unterworfen werden. Bei dieser Gelfiltration wird das Herbicolin in Herbicolin A und Herbicolin B getrennt. Die zuerst austretenden Herbicolin A enthaltenden Fraktionen werden gesammelt und in an sich bekannter Weise eingedampft oder der Gefriertrocknung unterworfen. Man erhält dabei reines Herbicolin A.

Die Herbicolin B enthaltenden Fraktionen werden ebenfalls gesammelt und eingedampft oder der Gefriertrocknung unterworfen. Man erhält hierbei reines Herbicolin B.

Alternativ kann die Herbicolin enthaltende Lösung zur Trennung des Herbicolins in Herbicolin A und Herbicolin B einer Craig-Verteilung unterworfen werden. Beispielsweise kann man für die Ober- und Unterphase für die Craig-Verteilung Gemische aus Butanol/Essigester/Wasser (1 : 2 : 1) verwenden. Bei einer Anlage mit 140 Stufen erhält man das Herbicolin A in den Stufen 60 bis 90.

Die Trennung von Herbicolin in Herbicolin A und Herbicolin B kann ebenfalls durch präparative Dünnschichtchromatographie erfolgen.

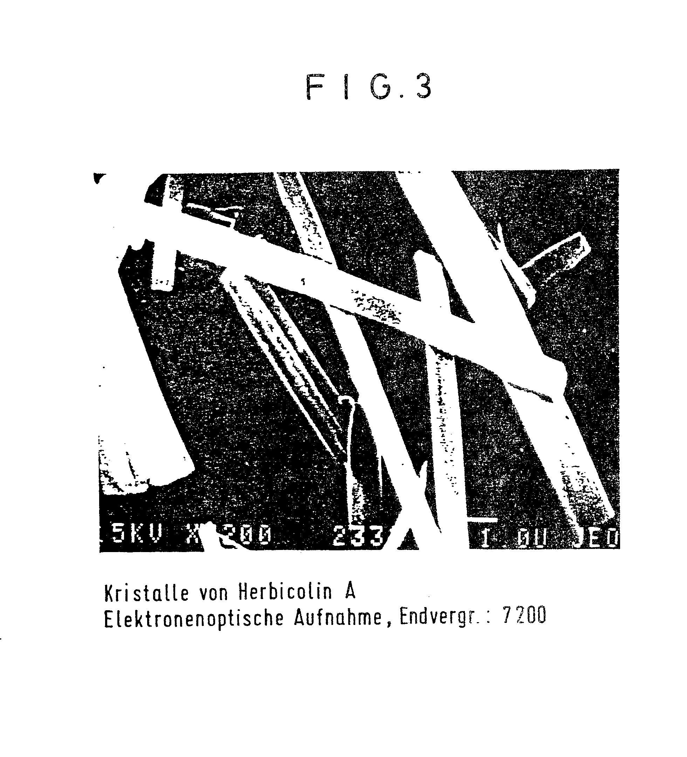

Nach Entfernung der Lösungsmittel in an sich bekannter Weise und/oder nach der Gefriertrocknung erhält man das Herbicolin, Herbicolin A oder Herbicolin B in hochgereinigter Form als weißes amorphes Pulver. Herbicolin A kann aus konzentrierten methanolischen Lösungen auch in kristalliner Form erhalten werden. Die Kristalle sind ca. 10 bis 50 μm lange feine Nadeln mit überwiegend rechteckigem Querschnitt; in der beigefügten Fig. 3 sind Kristalle von Herbicolin A in einer elektronenoptischen Aufnahme in einer Endvergrößerung von 7200 dargestellt.

Herbicolin, Herbicolin A und Herbicolin B besitzen im wesentlichen, wie oben angegeben, die gleiche antibiotische und antimykotische Wirkung.

Das erfindungsgemäße Antibiotikum Herbicolin A ist neu. Es kann durch folgende Angaben charakterisiert werden.

a) Löslichkeit und Eigenschaften:
Herbicolin A ist eine farblose, in Methanol, Äthanol, Propanol-(1) und Butanol-(1) sehr gut lösliche Substanz. Es ist gut löslich in Äthylenglykol, Äthylenglykolmonomethyläther, Glycerin und Wasser, wobei unter »gut löslich« verstanden wird, daß für die Erzielung der biologischen Wirkung genügend Herbicolin A in Lösung vorhanden ist.

In anderen Lösungsmitteln, wie Aceton, Chloroform, Dichlormethan, Acetonitril, Essigester und Petroläther, ist Herbicolin A nahezu unlöslich. Es zeigt eine negative Reaktion mit Ninhydrin, aber eine positive Reaktion mit Chlor/4,4'-Tetramethyldiamin-diphenylmethan.

b) Das Antibiotikum zeigt keine Absorption im UV zwischen 220 und 350 nm. Im IR-Spektrum treten Banden auf bei 3200, 2900, 2830, 1730, 1650 und 1530 cm$^{-1}$. In der beigefügten Fig. 1 ist das IR-Spektrum dargestellt.

c) Das Herbicolin A zeigt in $^{12}CD_3OD$ ein charakteristisches $^{13}C$-NMR-Spektrum. In den beigefügten Fig. 2a und 2b ist das NMR-Spektrum von Herbicolin A dargestellt.

d) Die Aminosäureanalyse mit Hilfe eines Aminosäureanalysators ergibt fünf Aminosäuren:
Glycin, Threonin, Leucin, Glutaminsäure und Arginin in einem Verhältnis von 2 : 2 : 1 : 1 : 1.

Es ist anzunehmen, daß der Peptidrest von Herbicolin A ein Heptapeptid ist, das zyklisch oder linear sein kann. Eine $\alpha$-Helix-Konformation des Peptidteils kann durch CD-Messungen ausgeschlossen werden.

e)  Dünnschichtchromatographie auf Kieselgelplatten:
   Der Antibiotikumproduzent bildet zwei biologisch aktive Antibiotika, Herbicolin A (ca. 80%) und Herbicolin B (ca.20%), die in der Dünnschichtchromatographie im Fließmittel 1-Butanol/Eisessig/Wasser (4 : 1 : 1) zwei Flecken ergeben: Herbicolin A (Rf = 0,06) und Herbicolin B (RF = 0,19).Im Fließmittel Chloroform/Methanol/Wasser (65 : 25 : 4) erhält man ähnliche Rf-Werte. Die Herbicoline werden durch Behandlung mit Chlor/4,4'-Tetramethyldiamindiphenylmethan oder durch Besprühen mit Wasser sichtbar gemacht.

f)  Die üblichen Zucker konnten nicht nachgewiesen werden. Die nichtpeptidischen Komponenten sind vermutlich keine Kohlenhydrate. Ein aromatischer oder heteroaromatischer Rest des Herbicolins A kann ebenfalls durch das UV-Spektrum ausgeschlossen werden, das keine Absorptionsbande im Bereich von 220 bis 320 nm aufweist.

g)  Die vollständige Struktur von Herbicolin A ist noch unbekannt.

Der erfindungsgemäße Wirkstoff Herbicolin zeigt eine starke fungitoxische Wirkung bei Konzentrationen von 0,1 bis µg/ml und verursacht Deformationen der Hyphenspitzen.

Daneben ist der Wirkstoff gegen Protozoen wirksam. Parameciumarten (Pantoffeltierchen) und Amöben werden in Konzentrationen von 1 bis 2 µg/ml in wenigen Sekunden abgetötet.

Erythrozyten werden in Konzentrationen von ca. 10 µg/ml (Humanblut) lysiert.

Grünalgen, wie z. B. Chlorella und Scenedesmus reagieren sehr unterschiedlich. Sie sind jedoch im allgemeinen widerstandsfähiger.

Alle Untersuchungen zum Wirkort deuten darauf hin, daß Herbicolin aufdie Cytoplasmamembran eukaryontischer Organismen wirkt. Hierbei sind die einzelnen Organismengruppen in unterschiedlich starkem Maße empfindlich.

Herbicolin A kristallisiert aus methanolischer Lösung in Form von ca. 10 bis 50 µm langer feiner Nadeln mit überwiegend rechteckigem Querschnitt.

Die folgenden Beispiele erläutern die Erfindung.

Beispiel 1

a) Herstellung von Herbicolin

Zur Gewinnung von Herbicolin wird ein chemisch definiertes Medium angesetzt, welches pro Liter folgende Bestandteile enthält: 7 kg $K_2HPO_4$, 3 g $KH_2PO_4$, 0,5 g tri-Na-Citrat · 2 $H^2O$, 0,1 g $MgSO_4$ 7 $H_2O$, 2 g $(NH_4)_2SO_4$ und 20 g Glucose (getrennt autoklaviert). Die eingewogenen Substanzen werden mit destilliertem oder entionisiertem Wasser im gewünschten Volumen gelöst, auf pH 6,8 eingestellt und im Autoklaven sterilisiert.

Ein 500-ml-Erlenmeyerkolben mit seitlichem Einstich, der 100 ml Nährlösung enthält, wird mit dem Produzentenstamm »Erwinia herbicola(A 111)« beimpft und 24 h bei 22°C auf einer rotierenden Schüttelmaschine bei 120 Umdrehungen/min inkubiert. Mit dieser Vorkultur wird ein 5-l-Erlenmeyerkolben mit seitlichem Einstich, der 2 l Nährmedium enthält, beimpft und 48 h bei 22°C mit 110 Umdrehungen/min inkubiert. Mit dieser zweiten Vorkultur werden fünf weitere 5-l-Erlenmeyerkolben mit je 2 l Nährlösung beimpft und drei Tage bei 22°C und 110 Umdrehungen/min inkubiert.

Die Bakterien werden durch Zentrifugation (Sorvall, Rotor GSA) 20 min bei 6 000 xg oder durch kontinuierliche Trennung (Sorvall, Rotor SZ-14 GK) abgetrennt.Die bakterienfreie Kulturflüssigkeit wird über eine Servachrom-XAD-2-Säule (60 × 500 mm) gegeben. Das Eluat wird verworfen. Danach wird mit fünf Säulenvolumen entionisiertem Wasser eluiert, um alle nichtadsorbierenden, wasserlöslichen Komponenten zu entfernen. Herbicolin verbleibt mit anderen überwiegend lipophilen Komponenten auf der Säule. Ein Teil der lipophilen Begleitkomponenten sowie der größte Teil der gelben Pigmente lassen sich durch eine Elution mit Aceton/Wasser (1 : 1) entfernen. Das Elutionsvolumen sollte ein Säulenvolumen nicht übersteigen, da auch schon geringe Mengen an Herbicolin miteluiert werden.

Herbicolin wird mit einemSäulenvolumen reinem Methanol von der Säule desorbiert. Das erhaltene Eluat wird am Rotationsverdampfer auf ein kleines Volumen eingeengt und auf eine Sephadex-LH-20-Säule (50 × 150 mm) gegeben. Durch Elution mit reinem Methanol kann die Herbicolin-Fraktion von anderen Begleitkomponenten abgetrennt werden.

Nach Entfernen des Lösungsmittels und anschließender Gefriertrocknung erhält man Herbicolin als weißes amorphes Pulver.

b) Herstellung von Herbicolin A

Zur Herstellung von Herbicolin A wird entweder das Eluat von der XAD-Säule oder die weiter gereinigte Fraktion von der Sephadex-LH-20-Säule einer Craigverteilung unterworfen. Ober- und Unterphase werden aus Butanol/Essigester/ Wasser (1 : 1 : 1) hergestellt. Die zu reinigende Herbicolinfraktion wird in der Oberphase gelöst. Bei einer Anlage von 140 Stufen erhält man reines Herbicolin A in den Stufen 60 bis 90. Die Fraktionen werden zur Trockene eingeengt und in wenig Methanol aufgenommen. Aus konzentrierten methanolischen Lösungen kann Herbicolin A in kristalliner Form gewonnen werden.

## Beispiel 2

### Wirkung von Herbicolin A gegen Neurospora crassa 74 A im Plattendiffusionstest

Man stellt 100 ml Hefe/Malzagar für Neurospora crassa her, der 1,5% Agar, 0,4% Hefeextrakt, 1% Malzextrakt und 0,4% Glucose enthält. Nach dem Autoklavieren läßt man auf 45°C abkühlen und gibt 0,1 ml einer Sporenlösung (ca. $10^6$ Sporen/ml) hinzu. Nach gutem Verteilen der Sporen gießt man in sterile Petrischalen und läßt erkalten. Auf Filterplättchen mit 6 mm Durchmesser werden 1 µl, 2 µl, 3 µl ... bis 10 µl einer methanolischen Herbicolinlösung (1 mg/ml) gegeben. Nach 15 min Trocknung an der Luft werden diese auf den mit Pilzsporen beimpften Nährboden gelegt und bei 27°C im Brutschrank bebrütet.

Nach 18 h sind in Abhängigkeit von der eingesetzten Menge an Herbicolin unterschiedlich große wachstumsfreie Zonen (Hemmhöfe) um die Filterplättchen zu erkennen. Hemmhöfe werden sichtbar bei Konzentrationen von 1 bis 5 µg und erreichen einen Durchmesser von 15 bis 18 mm bei 10 µg/Plättchen.

## Beispiel 3

### Wirkung von Herbicolin A gegen Neurospora crassa 74 A in Flüssigkultur

Man stellt 2 l eines chemisch definierten Mediums für Neurospora crassa her, welches folgende Bestandteile pro Liter dest. Wasser enthält: 5 g Asparagin, 1 g $K_2HPO_4$ 3 $H_2O$, 1 g $MgSO_4$ 7 $H_2O$, 0,5 g $CaCl_2$ 2 $H_2O$, 0,01 mg $ZnSO_4$ 7 $H_2O$ und 2 g Glucose (getrennt autoklaviert). Nach dem Sterilisieren wird mit 2 × $10^6$ Sporen beimpft und auf 10 Schüttelkolben (à 200 ml) verteilt. Man gibt steigende Mengen (0, 0,1, 0,2, 0,3... bis 1,0 ml) einer methanolischen Herbicolin-A-Lösung (0,1 mg/ml) zu und inkubiert auf einer rotierenden Schüttelmaschine bei 27°C.

Nach 48 h wird von jedem Ansatz abfiltriert und das gebildete Mycel nach Trocknung bei 100°C (24 h) gravimetrisch bestimmt.

Als typisches Ergebnis seien folgende Werte aufgeführt:

Kontrolle (ohne Herbicolin A) 249,5 mg (=100% Wachstum)

| Herbicolin A | Mycel (Trockengewicht) | % der Kontrolle |
|---|---|---|
| 0,1 ml | — 204 mg | (96,2%) |
| 0,2 ml | — 79 mg | (31,7%) |
| 0,3 ml | — 26 mg | (10,4%) |
| 0,4 ml | — 5 mg | (2,0%) |
| 0,5 ml | — 2 mg | (1,0%) |
| 0,6 — 1 ml | 1 mg | |

Die minimale Hemmkonzentration liegt damit bei ca. 0,25 µm/ml.

## Beispiel 4

### Bestimmung des Wirkungsspektrums von Herbicolin A

Die zu prüfenden Pilze und Bakterien werden mit Zellzahlen von $10^6$ pro 100 ml Nähragar als Sporen oder vegetative Zellen im Gußplattenverfahren oder durch Ausspateln auf Petrischalen verteilt. Von einer methanolischen Herbicolin-A-Lösung werden je 10 µl auf Filterplättchen-(∅ 6 mm) pipettiert, die nach dem Trocknen an der Luft auf die Petrischalen mit den verschiedenen Mikroorganismen gebracht werden. Zur Testung der Pilze wird Hefe/Malzagar und zur Testung der Bakterien Müller-Hinton-Agar verwendet. Als positives Ergebnis (Hemmung) wird gewertet, wenn Hemmhöfe größer als 9 mm sichtbar werden.

Das so erhaltene Wirkungsspektrum geht aus Tabelle I hervor.

Tabelle I

Wirkungsspektrum von Herbicolin A

| | |
|---|---|
| Aspergillus fumigatus | + |
| Aspergillus melleus | + |
| Botrytis cinerea | + |
| Fusarium dimerum | + |
| Gliocladium vermoeseni | + |
| Paecilomyces varioti | + |
| Neurospora crassa | + |
| Cryptococcus melibiosum | + |
| Candida guilliermondii | + |
| Candida albicans | + |
| Candida parapsilosis | + |
| Candida crusei | + |
| Schinzonella melanogramma | + |
| Urocystis occulta | + |
| Ustilago nuda | + |
| Trichophyton rubrum | + |
| Epidermophyton floccosum | + |

Tabelle I (Fortsetzung)

| | |
|---|---|
| Bacillus subtilis | — |
| Enterobacter cloacae | — |
| Escherichia coli | — |
| Klebsiella pneumoniae | — |
| Mycoplasma laidlawii | — |
| Proteus mirabilis | — |
| Proteus vulgaris | — |
| Pseudomonas aeruginosa | — |
| Staphylococcus auereus | — |
| Staphylococcus epidermitis | — |
| Streptococcus pyogenes | — |

Beispiel 5

Vergleich der antifungischen Aktivität
von Herbicolin A
mit anderen antifungischen Agentien

1) Um die Wirkung von Herbicolin A mit anderen bekannten antifungischen Substanzen zu demonstrieren, wird ein vergleichender Plattendiffusionstest mit Griseofulvin, Amphotericin und Herbicolin A und Neurospora crassa als Testkeim durchgeführt. Jeweils 10 µg der entsprechenden Antibiotika werden als methanolische Lösungen auf die Filterplättchen pipettiert und nach dem Trocknen auf einen mit Neurospora-crassa-Sporen beimpften Nährboden gelegt. Man erhält folgendes Ergebnis:Nach 18 h Bebrütung bei 27°C wird mit Griseofulvin kein Hemmhof, mit Amphotericin ca. 10 mm Hemmhof und mit Herbicolin A ca. 18 mm Hemmhof beobachtet.

2) In der gleichen Weise werden verschiedene membranmodifizierende Peptidantibiotika vergleichend mit Herbicolin A getestet: Trichotoxin A 40, Suzukazillin, Alamethicin F 50 und Herbicolin A, je 10 µg/Plättchen.Man erhält folgendes Ergebnis:Trichotoxin zeigt bei dieser Konzentration keinen, Suzukazillinund Alamethicin nureinen schwachen, Herbicolin A dagegen einen deutlich sichtbaren Hemmhof.

**Patentansprüche**

1. Herbicolin, bestehend aus Herbicolin A und Herbicolin B, erhalten durch Züchtung von Erwinia herbicola (A 111)(DSM. Nr. 1619) unter aeroben Bedingungen in einem Kohlenstoffquellen und Stickstoffquellen enthaltenden Kulturmedium und Gewinnung des Antibiotikums.
2. Herbicolin A, gekennzeichnet durch

1) charakteristische IR-Banden in Kaliumbromid (Fig. 1) bei 3200, 2900,2830, 1730, 1650, 1530 cm$^{-1}$

2) Löslichkeit:
sehr gut löslich in
    Methanol
    Äthanol
    Propanol-(1)
    Butanol-(1)
gut löslich in
    Äthylenglycol
    Äthylenglycolmonomethyläther
    Glycerin
    Wasser
gering löslich in
    Aceton
    Essigester
    Acetonnitril
nahezu unlöslich in
    Chloroform
    Dichlormethan
    Diäthyläther
    Petroläther

wobei unter »gut löslich« verstanden wird, daß für die Erzielung der biologischen Wirkung genügend Herbicolin A in Lösung vorhanden ist,

3) Elementeranalyse: Gef.: C 46,1, H 7,1 N 11,8 S 1,9 Molmasse 1500 bis 3000

4) Charakteristische Reaktionen:
Ninhydrin negativ
Chlor/4,4'-Tetramethyldiamindiphenylmethan positiv

5) keine Absorption in UV zwischen 220 und 350 nm

6) nachweisbare Aminosäuren:

| Aminosäuren | Verhältnis (Näherung) |
|---|---|
| Glycin | 2 |
| L-Threonin | 1 |
| D-allo-Threonin | 1 |
| D-Glutaminsäure | 1 |
| D-Leucin | 1 |
| L-Arginin | 1 |

6a) $\beta$-Hydroxymyristinsäure

7) $^{13}$C-NMR Spektrum (Fig. 2a und 2b) mit ppm ($\varepsilon$)-Werten in $^{12}CD_3OD$ von 177,7, 175,1, 174,5, 173,3 [173,6], 172,9, 172,5, 171,2, 170,9, 170,8,169,8, 167,9, 158,3, 132,5, 122,4, 97,9, 74,9,73,8, 73,8, 72,0, 71,5, 69,7, 68,8, 62,5, 61,2, 57,4, 54,6, 53,8, 53,2, 44,8, 44,1, 42,7, 40,8,

38,4, 33,0, 32,4, 32,0, 30,7, 30,4, 26,6, 25,9, 23,6, 21,98 [21,87], 17,6, 16,2 und 14,4

8) folgende Rf-Werte:
Butanol-(1)/Eisessig/Wasser
(4 : 1 : 1) = Rf 0,06
Chloroform/Methanol/Eisessig/Wasser
(65 : 25 : 3 : 4) = Rf 0,10

9) Wirksamkeit: starke fungitoxische Wirkung bei Konzentrationen von 0,1 bis 1 µg/ml Parameciumarten (Pantoffeltierchen) und Amöben werden in Konzentrationen von 1 bis 2 µg/ml in wenigen Sekunden abgetötet Erythrozyten werden in Konzentrationen von ca. 10 µg/ml (Humanblut) lysiert.

3. Herbicolin B, gekennzeichnet durch

a) charakteristische Reaktionen:
Chlor/4,4'-Tetramethyldiamindiphenylmethan positiv

b) Dünnschichtchromatographie
auf Kieselgelplatten:
Butanol-(1)/Eisessig/Wasser
(4 : 1 : 1) = Rf 0,19.

4. Verfahren zur Herstellung von Herbicolin, dadurch gekennzeichnet, daß man Erwinia herbicola (A 111) (DSM Nr. 1619) unter aeroben Bedingungen in einem Kohlenstoffquellen und Stickstoffquellen enthaltenden Kulturmedium züchtet und die Kulturlösung durch Zentrifugieren von den Bakterien befreit, das Zentrifugat auf eine Servachrom-XAD®-Säule gibt, mit entionisiertem Wasser wäscht, uur Entfernung von Pigmenten oder anderen lipophilen Komponenten mit Aceton/Wasser (1 : 1) oder Lösungsmittelgemischen ähnlicher Polarität wäscht und dann das gewünschte Herbicolin mit Methanol oder einem Lösungsmittel ähnlicher Polarität eluiert und das erhaltene Eluat gegebenenfalls zur Gewinnung von Herbicolin eindampft und gegebenenfalls gefriertrocket.

5. Verfahren nach Anspruch 4, dadurch gekennzeichnet, daß die Züchtung bei einem pH-Wert von 5,5 bis 8 durchgeführt wird.

6. Verfahren nach Anspruch 4 oder 5, dadurch gekennzeichnet, daß die Züchtung bei einer Temperatur zwischen 4 und 30°C durchgeführt wird.

7. Verfahren zur Gewinnung von Herbicolin A und Herbicolin B, dadurch gekennzeichnet, daß man das gemäß dem Verfahren nach Anspruch 4 erhaltene Eluat der Servachrom-XAD®-Säule durch Gelfiltration an Sephadex Lh 20® mit Methanol oder einem Lösungsmittel ähnlicher Polarität an Sephacryl 200 oder an Biogel P 4® in Methanol/Wasser (1 : 1) oder Aceton/Wasser (1 : 1) oder Lösungsmitteln ähnlicher Polarität behandelt und man die Herbicolin A und Herbicolin B enthaltenden Fraktionen getrennt sammelt, wobei das Herbicolin A zuerst eluiert wird.

8. Verfahren zur Gewinnung von Herbicolin A und Herbicolin B, dadurch gekennzeichnet, daß man das gemäß dem Verfahren nach Anspruch 7 erhaltene Eluat der Servachrom-XAD®-Säule durch Craig-Verteilung oder präparative Dünnschichtchromatographie in Herbicolin A und Herbicolin B auftrennt.

9. Verfahren nach Anspruch 8, dadurch gekennzeichnet, daß bei der Craig-Verteilung für die Ober- und Unterphase ein Gemisch aus Butanol/Essigester/Wasser (1 : 1 : 1) verwendet wird und man die Craig-Verteilung mit 140 Stufen anlegt und das Herbicolin A in den Stufen 60 bis 90 gewinnt.

10. Verfahren zur Gewinnung von reinem Herbicolin A, dadurch gekennzeichnet, daß man die bei Anspruch 7, 8 oder 9 Herbicolin A enthaltende Lösung zur Gewinnung von reinem Herbicolin A eindampft und/oder einer Gefriertrocknung unterwirft.

11. Verfahren zur Gewinnung von reinem Herbicolin B, dadurch gekennzeichnet, daß man die bei Anspruch 7, 8 oder 9 Herbicolin B enthaltende Lösung zur Gewinnung von reinem Herbicolin B eindampft und/oder einer Gefriertrocknung unterwirft.

12. Antibiotisches und antimykotisches Mittel, dadurch gekennzeichnet, daß es Herbicolin nach Anspruch 1 sowie übliche Trägerstoffe und Verdünnungsmittel enthält.

13. Antibiotisches und antimykotisches Mittel, dadurch gekennzeichnet, daß es Herbicolin A nach Anspruch 2 sowie übliche Trägerstoffe und Verdünnungsmittel enthält.

14. Antibiotisches und antimykotisches Mittel, dadurch gekennzeichnet, daß es Herbicolin B nach Anspruch 3 sowie übliche Zusatzstoffe und Verdünnungsmittel enthält.

**Claims**

1. Herbicolin, consisting of herbicolin A and herbicolin B, obtained by culturing Erwinia herbicola (A 111) (DSM. No. 1619) under aerobic conditions in a culture medium containing carbon sources and nitrogen sources and recovering the antibiotic.

2. Herbicolin A, characterized by

1) characteristic IR-bands in potassium bromide (Fig. 1) at 3200, 2900, 2830, 1730, 1650, 1530 cm$^{-1}$

2) solubility:
highly soluble in
methanol
ethanol
l-propanol
1-butanol
readily soluble in
ethylene glycol
ethylene glycolmonomethyl ether
glycerol
water

sparingly soluble in
    acetone
    ethyl acetate
    acetonitrile
substantially insoluble in
    chloroform
    dichloromethane
    diethyl ether
    petroleum ether,

»readily soluble« being understood to mean that sufficient herbicolin A is present in solution to obtain the biological effect;

3)   elemental analysis:
    observed:C 46.1, H 7.1 N 11.8, S 1.9
    molecular weight 1500 to 3000

4)   characteristic reactions:
    ninhydrin         negative
    chlorine/4,4'-tetramethyldiamine
    diphenyl methane    positive

5)   no UV-absorption between 220 and 350 nm

6)   detectable amino acids:

| Aminoacids | ratio (approx.) |
|---|---|
| glycine | 2 |
| L-threonine | 1 |
| D-allo-threonine | 1 |
| D-glutamic acid | 1 |
| D-leucine | 1 |
| L-arginine | 1 |

6a)   $\beta$-hydroxymyristic acid

7)   $^{13}$C-NMR spectrum (Figs 2a and 2b) with ppm ($\varepsilon$)-values in [12]CD$_3$OD of 177.7, 175.1, 174.5, 173.3 [173.6], 172.9,172.5, 171.2, 170.9, 170.8, 169.8, 167.9, 158.3, 132.5, 122.4, 97.9, 74.9, 73.8, 73.1, 72.0, 71.5, 69.7, 68.8, 62.5, 61.2, 57.4, 54.6, 53.8, 53.2, 44.8, 44.1, 42.7, 40.8, 38.4, 33.0, 32.4, 32.0, 30.7, 30.4, 26.6, 25.6, 23.6, 21.98, [21.87], 17.6, 16.2 and 14.4

8)   the following Rf-values:
    1-butanol/glacial acetic acid/water
    (4 : 1 : 1)         = Rf 0.06
    chloroform/methanol/glacial acetic
    acid/water
    (65 : 25 : 3 : 4)     = Rf 0.10

9)   activity:
    strong fungitoxic effect in concentrations of from 0.1 to 1 μ g/ml
paramecium species(slipper animalcules) and amoebae are destroyed in a few seconds by concentrations of from1 to 2 μg/l erythrocytes are lyzed in concentrations of approximately10 μg/ml (human blood).

3. Herbicolin B, characterized by

a)   characteristic reactions:
    chlorine/4,4'-tetramethyl diamine
    diphenyl methane    positive

b)   thin-layer chromatography on silica gel plates: 1-butanol/glacialacetic acid/water (4 : 1 : 1)         = Rf 0.19.

4. A process for the production of herbicolin, characterized in that Erwinia herbicola (A 111) (DSM No. 1619) is cultured under aerobic conditions in a culture medium containing carbon sources and nitrogen sources and the culture solution is freed from the bacteria by centrifuging, the centrifugate is poured onto a Servachrom-XAD®-column, washed with deionized water, followed by washing with acetone/water (1 : 1) or solvent mixtures of similar polarity to remove pigments and other lipophilic components, after which the required herbicolin is eluted with methanol or a solvent of similar polarity and the eluate obtained is optionally concentrated by evaporation and optionally freeze-dried to recover herbicolin.

5. A processas claimed in Claim 4, characterized in that culturing is carried out at a pH-value of from 5.5 to 8.

6. A process as claimed in Claim 4 or 5, characterized in that culturing is carried out at a temperature of from 4 to 30° C.

7. A process for recovering herbicolin A and herbicolin B, characterized in that the eluate of the Servachrom-XAD®-column obtained in the process claimed in Claim 4 is treated by gel filtration on Sephadex LH 20® with methanol or with a solvent mixture of similar polarity on Sephacryl 200 or on Biogel P 4® in methanol/water (1 : 1) or acetone/water (1 : 1) or solvents of similar polarity and the fractions containing herbicolin A and herbicolin B are separately collected, the herbicolin A being eluted first.

8. A process for recovering herbicolin A and herbicolin B,characterized in that the eluate of the Servachrom-XAD®-column obtained in the process claimed in Claim 7 is separated up into herbicolin A and herbicolin B by Craig separation or by preparative thin-layer chromatography.

9. A process as claimed in Claim 8,characterized in that, in the Craig separation, a mixture of butanol, ethyl acetate and water (1 : 1 : 1) is used for the upper and lower phases and in that the Craig separation is carried out over 140 stages, the herbicolin 4 being obtained in stages 60 to 90.

10. A process for recovering pure herbicolin A, characterized in that the solution containing herbicolin A in Claim 7, 8 or 9 is concentrated and/or freeze-dried to recover pure herbicolin A.

11. A process for recovering pure herbicolin B, characterized in that the solution containing herbicolin B in Claim 7, 8 or 9 is concentrated by evaporation and/or freeze-dried to recover herbicolin B.

12. An antibiotic and antimycotic agent, characterized in that it contains the herbicolin claimed in Claim 1 together with standard excipients and diluents.

13. An antiobitic and antimycotic agent, characterized in that it contains the herbicolin A claimed in Claim 2 together with standard excipients and diluents.

14. An antibiotic and antimycotic agent, characterized in that it contains the herbicolin B in Claim 3 together with standard excipients and diluents.

**Revendications**

1. Herbicoline, formée d'herbicoline A et d'herbicoline B, obtenue par culture d'Erwinia herbicola (A 111) (DSM n° 1619) dans des conditions aérobies dans un milieu de culture contenant des sources decarbone et des sources d'azote, et par récupération de l'antibiotique.

2. Herbicoline A, caractérisée par:

1) des bandes IR caractéristiques dans le bromure de potassium, à 3200, 2900, 2830,1730, 1650,1530 cm$^{-1}$

2) sa solubilité:
très soluble dans le
    méthanol,
    l'éthanol,
    le propanol-(1),
    le butanol-(1)
soluble dans
    l'éthylèneglycol,
    l'éther monométhylique
    de l'éthylèneglycol,
    le glycérol,
    l'eau
peu soluble dans
    l'acétone,
    l'acétate d'éthyl,
    l'acétonitrile
à peu près soluble dans
    le chloroforme,
    le dichlororméthane,
    l'éther diéthylique,
    l'éther de pétrole,
»soluble« voulant dire ici qu'il y a en solution suffisamment d'herbicoline pour obtenir l'effet biologique,

3) son analyse élémentaire:
trouvé: C 46,1 H 7,1 N 11,8 S 1,9, masse moléculaire 1500 à 3000,

4) ses réactions caractéristiques
    ninhydrine          négative
    chloro-(4,4'-tétraméthyldiamino-
    diphénylméthane)   positive

5) aucune absorption dans l'UV entre 220 et 350 nm

6) aminoacides décelables:

| Aminoacides | (Approximation) |
| --- | --- |
| glycine | 2 |
| L-thréonine | 1 |
| D-allothréonine | 1 |
| acide D-glutamique | 1 |
| D-leucine | 1 |
| L-arginine | 1 |

6a) acide β-hydroxymyristique

7) son spectre RMN de $^{13}$C présentant,dans $^{12}$CD$_3$OD, des valeurs ($\varepsilon$), en ppm, de 177,7, 175,1, 174,5, 173,3 [173,6], 172,9, 172,5, 171,2, 170,9, 170,8, 169,8, 167,9, 158,3, 132,5, 122,4, 97,9, 74,9, 73,8, 73,1, 72,0, 71,5, 69,7, 68,8, 62,5, 61,2, 57,4, 54,6, 53,8, 53,2, 44,8, 44,1, 42,7, 40,8,38,4, 33,0, 32,4, 32,0, 30,7, 30,4, 26,6, 25,9, 23,6, 21,98 [21,87] 17,6, 16,2 et 14,4.

8) les valeurs Rf suivantes:
butanol-(1)/acide acétique glacial/eau
(4 : 1 : 1)          = Rf 0,06
chloroforme/méthanol/acide acétique/eau
(65 : 25 : 3 : 4)      = Rf 0,10

9) activité: forte action fongitoxique à des concentrations de 0,1 à 1 µg/ml des variétés de paramécie et des amibes sont tuées en quelques secondes dans des concentrations de 1 à 2µg/ml; les érythrocytes sont lysés à des concentrations d'environ 10 µg/ml (sang humain).

3. Herbicoline B, caractérisé par:

a) réactions caractéristiques:
chloro-(4,4'-tétraméthyldiaminodiphénylméthane)       positif

b) chromatographie en couche mince sur plaques de gel de silice: butanol-(1)/acide acétique glacial/eau
(4 : 1 : 1)          = Rf 0,19.

4. Procédé de préparation d'herbicoline, caractérisé en ce que l'on cultive Erwinia herbicola (A 111) (DSM n° 1619) dans des conditions aérobies, dans un milieu de culture contenant des sources de carbone et des sources d'azote et que l'on débarrasse la solution de culture des bactéries par centrifugation, que l'on verse le produit de centrifugation sur une colonne de Servachrom-XAD(marque déposée), qu'on le lave à l'eau désionisée, que pour éliminer les pigments et autres constituants lipophiles on le lave avec le mélange acétone/eau (1 : 1) ou avec des mélanges de solvants de polarité analogue, qu'ensuite on élue l'herbicoline désirée avec du méthanol ou un solvant de polarité analogue et qu'éventuellement on concentre par évaporation l'éluat obtenu pour obtenir l'herbicoline et qu'éventuellement on le lyophilise.

5. Procédé selon la revendication 4, caractérisé en ce que l'on effectue la culture à un pH de 5,5à 8.

6. Procédé selon l'une quelconque des revendications 4 et 5, caractérisé en ce que l'on effectue la culture à une température comprise entre 4 et 30°C.

7. Procédé d'obtention d'herbicoline A et d'herbicoline B, caractérisé en ce que l'on traite l'éluat de la colonne de Servachrom-XAD (marque déposée), obtenu selon le procédé selon la revendication 4, par filtration sur gel de Sephadex LH 20 (marque déposée), qu'on le traite par le méthanol ou un solvant de polarité analogue sur du Sephacryl 200 ou sur du Biogel P 4 (marque déposée) dans un mélange méthanol/eau (1 : 1)ou acétone/eau (1 : 1) ou des solvants de polarité analogue et que l'on recueille séparément les fractions contenant de l'herbicoline A et de l'herbicoline B, l'herbicoline A étant éluée en premier lieu.

8. Procédé d'obtention d'herbicoline A et d'herbicoline B, caractérisé en ce que l'on sépare en herbicoline A et herbicoline B, par distribution de Craig ou par chromatographie préparative en couche mince, l'éluat de la colonne de Servachrom-XAD (marque déposée), obtenue selon le procédé selon la revendication 7.

9. Procédé d'obtention d'herbicoline A et d'herbicoline B selon la revendication 8, caractérisé en ce que lors de la distribution de Craig, on utilise pour les phases supérieure et intéfieure un mélange butanol/acétate d'ethyle/eau (1 : 1 : 1) et que l'on organise la distribution de Craig en 140 stades et que l'on récupère l'herbicoline A aux stades 60 à 90.

10.Procédé d'obtention d'herbicoline A pure, caractérisé en ce que l'on concentre par évaporation la solution obtenue selon l'une quelconque des revendications 7, 8 et 9 et contenant de l'herbicoline A, pour obtenir de l'herbicoline A pure, et/ou qu'on la soumet à une lyophilisation.

11. Procédé d'obtention d'herbicoline B pure, caractérisé en ce que l'on concentre par évaporation la solution obtenue selon l'une quelconque des revendications 7,8 et 9 et contenant de l'herbicoline B, pour obtenir de l'herbicoline B pure, et/ou qu'on la soumet à une lyophilisation.

12. Agent antibiotique et antimycosique caractérisé en ce qu'il contient del'herbicoline selon la revendication 1, ainsi que des excipients et diluants usuels.

13. Agent antibiotique et antimycosique caractérisé en ce qu'il contient de l'herbicoline A selon la revendication 2 ainsi que que des excipients et diluants usuels.

14. Agent antibiotique et antimycosique caractérisé en ce qu'il contient de l'herbicoline B selon la revendication 3 ainsi que des addititifs et diluants usuels.

F I G. 1

F I G. 2a

$^{13}$C-NMR Spektrum
Herbicolin A in $^{12}$CD$_3$OD

x 2

FIG. 2b

$^{13}C$-NMR Spektrum
Herbicolin A in $^{12}CD_3OD$
( δ-Werte )

# F I G. 3

Kristalle von Herbicolin A
Elektronenoptische Aufnahme, Endvergr.: 7200